# EUROPEAN PATENT APPLICATION

(11) **EP 4 470 475 A1**
(43) Date of publication of application: **04.12.2024**
(21) Application number: 22924301.9
(22) Date of filing: 09.08.2022
(51) Int. Cl.: A61B 10/00

(54) **SAMPLE COLLECTION STICK**

(30) Priority: 27.01.2022 KR 20220011938
(71) Applicant: Bionlifescience, Inc., Namyangju-si, Gyeonggi-do 12106 (KR)
(72) Inventor: GOH, Chang Wook, Namyangju-si Gyeonggi-do 12095 (KR)
(74) Representative: dompatent von Kreisler Selting Werner - Partnerschaft von Patent- und Rechtsanwälten mbB
(86) International application number: PCT/KR2022/011818
(87) International publication number: WO 2023/146046

(57) **Abstract**

The present invention relates to a sample collection stick comprising a support part and a sample collection part, wherein, according to the present invention, the sample collection part has a cylindrical shape in which an accommodation space that can accommodate a sample is provided, the accommodation space being arranged at the central axis in the length direction of the sample collection part, and a plurality of sample inlet/outlets that are in communication with the accommodation space are disposed at the outer surface of the sample collection part, the sample inlet/outlets being disposed in a line in the lengthwise direction of the sample collection part, and thus it is possible to increase the amount of sample collected from a subject of examination, and the amount of the collected sample that is dissolved or dispersed in a reagent or solution, from the sample collection stick, can be increased.

## Description

### [Technical Field]

The present invention relates to a sample collection stick, and more particularly, to a sample collection stick configured to collect a sample such as saliva from an oral cavity or a nasal cavity of a test subject and to collect the sample by scraping the saliva from the oral cavity or the nasal cavity of the test subject.

### [Background Art]

A microbiological test is performed to determine whether a sample from a test subject is contaminated with pathogens that may cause a disease. That is, when it is determined that a disease is caused by microorganisms, the microbiological test is performed to establish diagnosis, treatment, and prevention measures. Specifically, the microbiological test is performed to determine whether the test subject is contaminated with pathogens that may cause a disease, such as E. coli, typhoid fever, staphylococcus, and pseudomonas bacteria.

Generally, the sample is collected from the body of the test subject so as to perform the microbiological test. Then, the collected sample is mixed with a reagent or a solution for testing. Thereafter, the microbiological test is performed to determine presence or absence of pathogens.

When a sample is collected from saliva, bodily fluids, or bodily tissues of the test subject, a medical instrument called a stick, a brush, or a swab is used to collect the sample. In other words, a sample collection swab is introduced into the body of a test subject so as to collect a sample, and the sample is placed on the sample collection swab. Then, the sample collection swab is withdrawn from the body of the test subject.

A conventional sample collection swab has a stick shape, and a circular-shaped collection part is formed at an end of the swab. The collection part of the swab has a fiber layer provided thereon and formed by attaching small-sized microfibers to the end of the swab. In this manner, a sample may permeate the fiber layer formed on the collection part of the conventional swab. Thereafter, an examiner may collect the sample by withdrawing the collection part of the swab from the body of a test subject.

Meanwhile, in order to allow saliva or the like to permeate the fiber layer, the conventional sample collection swab is required to remain inserted into an oral cavity or a nasal cavity of a test subject for a predetermined time or longer, which causes significant inconvenience to the test subject. Above all, a part of the fiber layer formed on the sample collection swab may be separated and detached from a collection part of the swab and may remain in the body of the test subject. It is a serious medical problem if a foreign substance such as a fiber layer remains in the body of the test subject. In addition, as another problem of the conventional sample collection swab, test accuracy frequently deteriorates due to the insufficient number of collected samples.

Accordingly, there is demand for development of a technique capable of solving the above-described problems.

### [Disclosure]

### [Technical Problem]

Therefore, the present invention has been made in view of the above problems, and it is an object of the present invention to provide a sample collection stick configured to increase the number of collected samples during a sample collection process, to shorten the time required for sample collection, and to prevent a fiber portion constituting a fiber layer of a conventional swab from being separated from the swab.

### [Technical Solution]

In accordance with the present invention, the above and other objects can be accomplished by the provision of a sample collection stick including a support part formed in a rod shape or a bar shape, the support part having a predetermined length, and a sample collection part located at a front end of the support part and configured to collect a sample in contact with a body of a test subject, wherein the sample collection part is formed in a cylindrical shape, and wherein the sample collection part includes an accommodation space formed in a longitudinal central axis of the sample collection part and configured to accommodate the sample therein, and a plurality of sample inlets/outlets formed on an outer surface of the sample collection part and configured to communicate with the accommodation space, wherein the sample inlets/outlets are arranged in a row in a longitudinal direction of the sample collection part.

The plurality of sample inlets/outlets may be respectively arranged in a first row and a second row, wherein the first row and the second row may be formed with the longitudinal central axis of the sample collection part interposed therebetween.

The sample inlets/outlets in the first row and the sample inlets/outlets in the second row may be alternately arranged in the longitudinal direction of the sample collection part.

The sample collection part may have one or more partition walls provided therein and formed to spatially divide the accommodation space into two or more spaces.

The sample collection part may have a sample inlet/outlet passage provided therein and formed to be narrowed or expanded in a direction traversing, from the sample inlets/outlets, the longitudinal central axis of the sample collection part.

The sample collection part may be made of a flexible material or an elastic material.

The support part may have a coupling jaw or a coupling protrusion formed to protrude from an outer surface thereof or a lid coupling groove formed in the outer surface, thereby enabling the outer surface of the support part to be fixedly coupled to a lid of a sample container configured to accommodate the sample collection part therein.

### [Advantageous effects]

A sample collection stick according to the present invention does not require a flocking process required in a manufacturing process of a conventional sample collection swab, thereby having an effect of reducing manufacturing costs of the sample collection stick. Additionally, a fiber layer is not formed on the sample collection stick, thereby having an effect of solving a conventional problem that a fiber layer is partially separated from a conventional sample collection swab and remains in the body of a test subject. In this manner, it is possible to reliably secure safety of the test subject.

Furthermore, the sample collection stick according to the present invention increases the number of samples collected from the body of the test subject. Additionally, the conventional sample collection swab requires absorption time during which the sample permeates the fiber layer, but the sample collection stick according to the present invention does not require the above-mentioned absorption time, thereby having an effect of shortening the time required for sample collection. As a result, the time required for sample collection may be shortened, discomfort of the test subject may be alleviated during sample collection, and test accuracy may be improved by increasing the number of collected samples.

### [Description of Drawings]

The above and other objects, features and other advantages of the present invention will be more clearly understood from the following detailed description taken in conjunction with the accompanying drawings, in which:
FIG. 1 is a perspective view schematically showing a sample collection stick according to an embodiment of the present invention;
FIG. 2 is a side view schematically showing a sample collection part of the sample collection stick according to the embodiment of the present invention;
FIG. 3 is a view showing the sample collection part of the sample collection stick according to the embodiment of the present invention;
FIG. 4 is a side cross-sectional view schematically showing the sample collection part of the sample collection stick according to the embodiment of the present invention;
FIG. 5 is a perspective view schematically showing a side cross section of the sample collection part of the sample collection stick according to the embodiment of the present invention;
FIG. 6 is a cross-sectional view schematically showing a cross section of the sample collection part of the sample collection stick according to the embodiment of the present invention; and
FIG. 7 is a side cross-sectional view schematically showing a modified sample collection part of the sample collection stick according to the embodiment of the present invention.

### [Best Mode]

The present invention may be modified in various ways and may have various embodiments, and specific embodiments will be described in detail with reference to the accompanying drawings. However, it should be understood that the present invention is not limited to the specific embodiments, and the specific embodiments include all modifications, equivalents, and substitutes that fall within the spirit and technical scope of the present invention. The embodiments are provided to more completely explain the present invention to those having average knowledge in the art. Therefore, the shapes of elements in the drawings may be exaggerated to emphasize a clearer description. A detailed description of related known configurations or functions incorporated herein will be omitted when it is determined that the detailed description thereof may unnecessarily obscure the gist of the present invention.

Meanwhile, in the present invention, terms such as "first" and/or "second" may be used to describe various components, but the components are not limited by the terms. The terms are used only for the purpose of distinguishing one component from other components.

Terms used in the present invention are only used to describe specific embodiments and are not intended to limit the present invention. In this specification, an expression in a singular form also includes the plural sense, unless clearly specified otherwise in context.

It should be understood that expressions such as "comprise" and "have" in this specification are intended to designate the presence of indicated features, numbers, steps, operations, components, parts, or combinations thereof, but do not exclude the presence or addition of one or more features, numbers, steps, operations, components, parts, or combinations thereof.

Hereinafter, the present invention will be described in detail with reference to the accompanying drawings so that those skilled in the art to which the present invention pertains may easily implement the present invention. However, the present invention may be implemented in various ways and is not limited to the embodiments described herein. Wherever possible, the same reference numbers will be used throughout the drawings to refer to the same or like parts.

Hereinafter, a sample collection stick according to an embodiment of the present invention will be described with reference to the drawings.

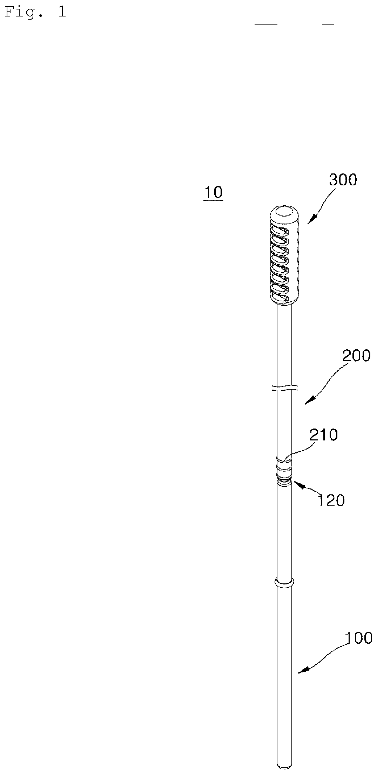

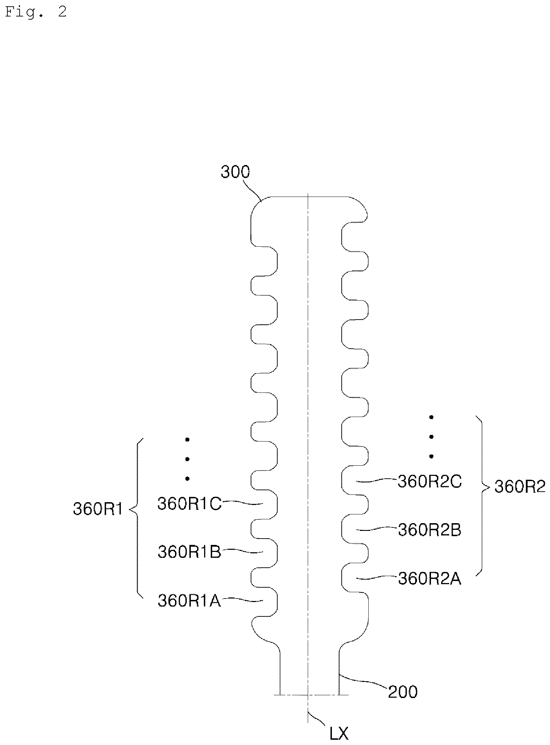

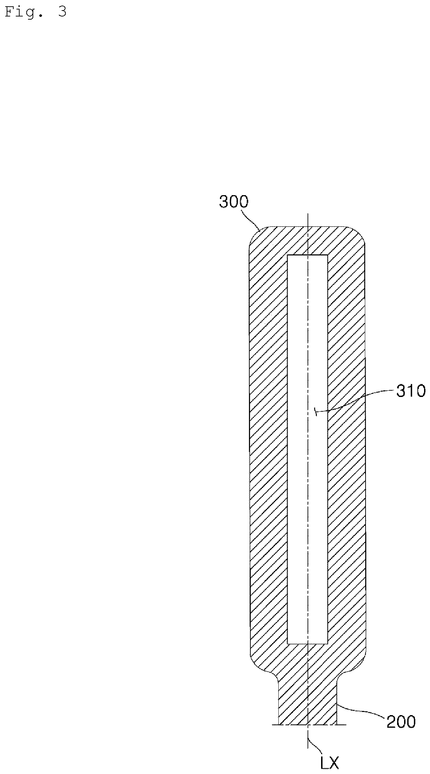

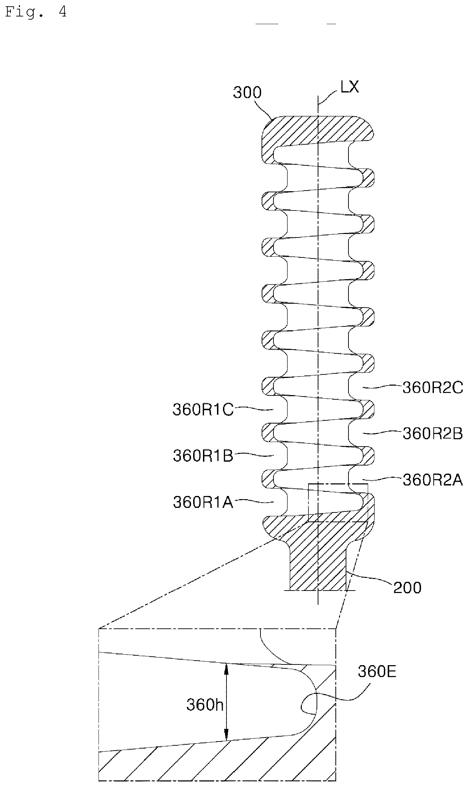

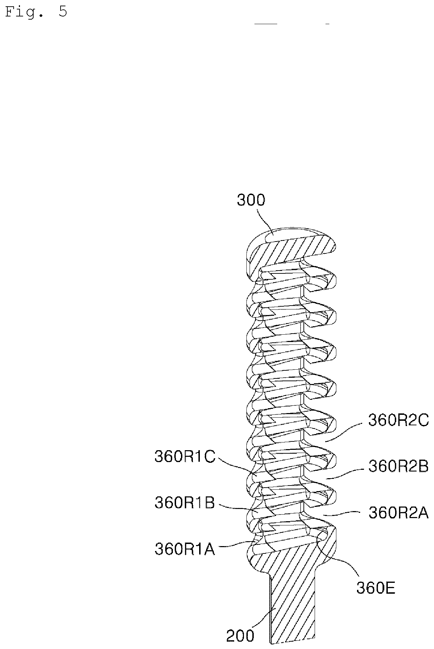

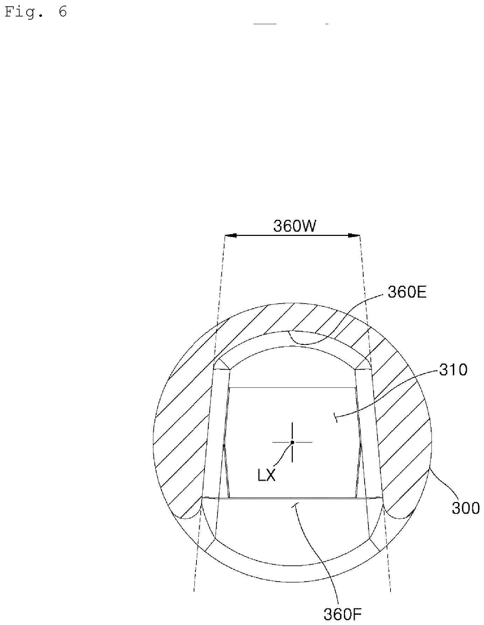

FIG. 1 is a perspective view schematically showing a sample collection stick according to an embodiment of the present invention, FIG. 2 is a side view schematically showing a sample collection part of the sample collection stick according to the embodiment of the present invention, FIG. 3 is a view showing the sample collection part of the sample collection stick according to the embodiment of the present invention, FIG. 4 is a side cross-sectional view schematically showing the sample collection part of the sample collection stick according to the embodiment of the present invention, FIG. 5 is a perspective view schematically showing a side cross section of the sample collection part of the sample collection stick according to the embodiment of the present invention, and FIG. 6 is a cross-sectional view schematically showing a cross section perpendicular to the longitudinal direction of the sample collection part of the sample collection stick according to the embodiment of the present invention.

First, as shown in FIG. 1, a sample collection stick 10 according to an embodiment of the present invention includes a support part 200, a sample collection part 300, and a handle part 100.

First, the support part 200 is formed in a rod shape or a bar shape and has a predetermined length. The sample collection part 300 is located on the front side of the support part 200 configured to support the sample collection part 300. The handle part 100 is connected to the rear side of the support part 200. The support part 200 supports the sample collection part 300 so as to enable the sample collection part 300 to enter the body of a test subject.

The front side of the support part 200 and the sample collection part 300 may be made of a single material such as a polymer material or an elastomer material. Alternatively, the support part 200 and the sample collection part 300 may be made of different materials, and the support part 200 and the sample collection part 300 may be combined with each other. In addition, it is sufficiently possible to fusion-mold the sample collection part 300 on the front side of the support part 200.

The support part 200 sufficiently supports the sample collection part 300 so as to enable the sample collection part 300 to enter the body of the test subject and to collect the sample from the body of the test subject. Here, the support part 200 is not limited to a specific form.

In addition, the support part 200 is preferably made of a polymer material having flexibility and elasticity. In this manner, the support part 200 may be flexibly deformed by force applied thereto from the outside and may return to an original shape thereof.

When a medical examiner withdraws, from a sample container (not shown), the sample collection part 300 and the support part 200 of the sample collection stick 10 using a tool such as tweezers, the medical examiner needs to clean the tweezers in advance. Then, after picking up the support part 200 of the sample collection stick 10 and withdrawing the sample collection stick 10 from the sample container with the tweezers, the medical examiner places the sample collection part 300 and the support part 200 of the sample collection stick 10 at a desired position. Thereafter, the tweezers need to be cleaned again.

In this manner, the tweezers used in the process of withdrawing the sample collection stick 10 from the sample container need to be washed each time, which may cause deterioration in efficiency of sample testing. In consideration of the above-described inconvenience, the following configuration may be provided so as to enable the sample collection part 300 and the support part 200 of the sample collection stick 10 to be withdrawn from the sample container without using the tweezers.

That is, the support part 200 may have a coupling jaw or a coupling protrusion 210 provided thereon and formed to protrude from the outer surface thereof, thereby enabling the outer surface of the support part 200 to be fixedly coupled to a cover or a lid of the sample container.

The cover or the lid (not shown) of the sample container (not shown) may have a fixing groove or an insertion jaw (not shown) provided on the inner side thereof and configured to allow the support part 200 of the sample collection stick 10 to be fixed to the cover or the lid of the sample container. That is, the sample container and the support part may be coupled to each other in such a manner that the fixing groove or the insertion jaw provided on the inner side of the cover or the lid of the sample container is engaged with or fitted into the coupling jaw or the coupling protrusion 210 of the support part.

As described above, the support part 200 may be fixed to the lid or the cover of the sample container. For example, after opening the lid or the cover of the sample container, a medical examiner may easily remove the support part 200 from the lid or the cover of the sample container without using a medical instrument such as tweezers.

FIG. 1 is a view showing an example of the coupling jaw or the coupling protrusion 210. In the example shown in FIG. 1, the coupling jaw or the coupling protrusion 210 is formed on the outer surface of the support part 200 in the circumferential direction of the support part 200 and has a ring shape. As shown in FIG. 1, one or more coupling jaws or one or more coupling protrusions each formed in the ring-like shape are preferably provided on the outer surface of the support part 200.

In this manner, the support part 200 is preferably coupled to the cover of the sample container, thereby improving convenience in use.

The handle part 100 is located on the rear side of the support part 200. That is, the rear end of the support part 200 is connected to the handle part 100.

The handle part 100 is connected to the rear end of the support part 200 and has a certain length. In this manner, the handle part 100 may be gripped by the hand or fingers of the medical examiner.

As shown in the drawing, the handle part 100 has a rod shape or a bar shape with a predetermined length. The handle part 100 is gripped by the hand or fingers of the medical examiner. The medical examiner may control the sample collection part 300 using the handle part 100, thereby placing or inserting the sample collection part 300 into the body of a test subject.

Accordingly, the sample collection part 300 may be placed or inserted into the body of the test subject through an oral cavity or a nasal cavity of the test subject by control of the medical examiner using the handle part 100.

As shown in the drawing, it is preferable to provide a segmental groove 120 such as a segmental joint, in which the segmental groove 120 is formed between the handle part 100 and the rear end of the support part 200 so as to distinguish the handle part 100 from the support part 200. That is, the segmental groove 120 is preferably provided between the handle part 100 and the support part 120, thereby separating the handle part 100 from the support part 200.

When the sample collection part 300 is withdrawn from the body of the test subject according to the intention of a user who is a medical examiner, the sample collection part 300 and the support part 200 are accommodated in the sample container.

Here, since there is no need to accommodate the handle part 100 in the sample container, it is preferable to separate the handle part 100 from the support part 200. Therefore, as shown in the drawing, it is preferable to provide the segmental joint or the segmental groove 120 between the support part 200 and the handle part 100, thereby enabling the support part 200 and the handle part 100 to be separated from each other according to the intention of the medical examiner.

After the support part 200 and the handle part 100 are separated from each other, the support part 200 may be coupled to the cover or the lid of the sample container as described above. In order to allow the sample collection part 300 to be fixedly coupled to the cover or the lid of the sample container, it is preferable to provide the protruding coupling jaw or the coupling protrusion 210 formed on the outer peripheral surface of the support part 200 or a lid coupling groove formed thereon.

The sample collection part 300 is a part that is inserted or placed into the body of a test subject and comes into contact with a sample, thereby performing sample collection. As shown in FIGs. 1 and 2, the sample collection part 300 is located at the front end of the support part 200 and comes into contact with the body of the subject so as to collect the sample from the test subject.

As shown in FIGs. 1 to 5, the sample collection part 300 is formed in a cylindrical shape and has an accommodation space 310 formed therein in the longitudinal central axis LX of the sample collection part 300 and configured to accommodate the sample from the test subject therein. The sample collection part 300 has a plurality of sample inlets/outlets 360R1 and 360R2 each configured to communicate with the accommodation space 310 and formed on the outer surface thereof. The plurality of sample inlets/outlets 360R1 and 360R2 may be arranged in a row in the longitudinal direction of the sample collection part 300.

That is, the sample collection part 300 formed in the cylindrical shape has the accommodation space 310 formed on the inner side thereof and configured to accommodate the sample therein, as shown in FIG. 3. In this case, when the plural sample inlets/outlets 360R1 and 360R2 are formed to cross the inner central axis LX from the outer surface of the sample collection part 300, as shown in FIG. 4 or FIG. 5, the plural sample inlets/outlets 360R1 and 360R2 each communicating with the accommodation space 310 are formed on the outer surface of the sample collection part 300.

Here, each of the sample inlets/outlets 360R1 and 360R2 is not formed in a hole shape straight passing through the longitudinal central axis LX of the sample collection part 300 in the vertical direction. As shown in FIGs. 4 to 6, each of the sample inlets/outlets 360R1 and 360R2 is formed to have an empty space starting from a corresponding one of the sample inlets/outlets 360R1 and 360R2 and crossing the central axis LX of the sample collection part. That is, each of the sample inlets/outlets 360R1 and 360R2 is preferably formed to have a groove 360E in which an end portion of an inlet/outlet passage for the sample is closed.

In this manner, each of the sample inlets/outlets 360R1 and 360R2 is not formed to have a hole straight passing through the sample collection part 300 but is formed to have a groove in the sample collection part 300, in which the groove has a closed end. Accordingly, the sample flowing into the sample inlets/outlets 360R1 and 360R2 may enter the accommodation space 310 and may be accommodated in the accommodation space 310 without passing through the opposite side of the accommodation space 310 and exiting from the accommodation space 310.

Preferably, the plural sample inlets/outlets 360R1 and 360R2 are formed on the outer surface of the sample collection part 300. As shown in the drawings, the plural sample inlets/outlets 360R1 and 360R2 are arranged in a row in the longitudinal direction of the sample collection part 300.

As an alternate description of the above-mentioned structural configuration in which the sample inlets/outlets 360R1 and 360R2 are arranged in a row in the longitudinal direction of the sample collection part 300, a plurality of sample inlets/outlets 360R1A, 360R1B, and 360R1C may be formed in a row from the rear side of the sample collection part 300 connected to the front end of the support part 200 to the front side of the sample collection part 300 in the longitudinal central axis LX.

Here, the plurality of sample inlets/outlets 360R1A, 360R1B, and 360R1C may be arranged in one row, that is, a first row.

In addition, a plurality of other sample inlets/outlets 360R2A, 360R2B, and 360R2C may be formed in a different row. Here, the plurality of other sample inlets/outlets 360R2A, 360R2B, and 360R2C may be arranged in the other row, that is, a second row.

As shown in FIG. 2 or FIG. 4, a direction in which the plural sample inlets/outlets 360R1 belonging to the first row are opened is preferably opposite a direction in which the plural sample inlets/outlets 360R2 belonging to the second row are opened.

In addition, the plural sample inlets/outlets 360R1 and the plural sample inlets/outlets 360R2 are respectively arranged in the first row and the second row, and the first row and the second row are preferably formed with the longitudinal central axis LX of the sample collection part 300 interposed therebetween.

Here, as shown in the drawings, the sample inlets/outlets 360R1 belonging to the first row and the sample inlets/outlets 360R2 belonging to the second row are preferably arranged alternately in the longitudinal direction of the sample collection part 300.

As described above, the sample inlets/outlets 360R1 in the first row and the sample inlets/outlets 360R2 in the second row are arranged alternately in the longitudinal direction of the sample collection part 300. Further, as described above, each of the sample inlets/outlets 360R1 and 360R2 is not formed to have a hole shape passing through the longitudinal central axis LX of the sample collection part 300 in the vertical direction. That is, the opposite sides of the sample inlets/outlets 360R1 and 360R2 are each formed to have the closed groove 360E.

Therefore, during the sample collection process, it is possible not only to prevent the sample flowing into the sample inlets/outlets 360R1 and 360R2 and entering the accommodation space 310 from passing through the central axis LX and exiting therefrom, but also to maintain a state in which the sample is accommodated in the accommodation space 310.

In addition, a sample inlet/outlet passage 360F is preferably formed to be narrowed or expanded in a direction in which the sample passes through the longitudinal central axis LX of the sample collection part 300 from each of the sample inlets/outlets 360R1 and 360R2. In other words, as the sample further enters from each of the sample inlets/outlets 360R1 and 360R2 to the inside of the accommodation space 310, the sample inlet/outlet passage 360F is preferably formed to be narrowed or expanded.

For example, as shown in FIGs. 4 and 6, a height 360h of the sample inlet/outlet passage 360F gradually decreases in a direction from the sample inlet/outlet 360R1A to the central axis LX, thereby obtaining a gradually narrowed sample inlet/outlet passage 360F.

In addition, a width 360W of the sample inlet/outlet passage 360F gradually decreases in a direction from the sample inlet/outlet 360R1A to the central axis LX, thereby obtaining a gradually narrowed sample inlet/outlet passage 360F.

In this manner, when the sample inlet/outlet passage 360F is formed to be gradually narrowed in a direction from each of the sample inlets/outlets 360R1 and 360R2 to the inner side of the accommodation space 310, it is possible to collect the sample that is introduced through each of the sample inlets/outlets 360R1 and 360R2 and enters the accommodation space 310 formed in the sample collection part 300.

The samples introduced into the sample inlet/outlet passage 360F or collected in the accommodation space 310 have cohesiveness, thereby making it possible to prevent the samples from being separated from the sample collection part 300 during the sample collection process.

When the sample collection part 300 is immersed in a fixative solution, the samples held or accommodated in the sample collection part 300 may be easily dispersed in the fixative solution, thereby increasing the number of collected samples in the fixative solution.

In other words, when the sample collection part 300 is immersed in the fixative solution, the fixative solution flows into the accommodation space 310. Accordingly, the accommodation space 310 is filled with the samples accommodated therein and the fixative solution, and some samples are dispersed in the fixative solution. In this state, when the sample collection part 300 is shaken by shaking the support part 200, the samples are further dispersed in the fixative solution, thereby allowing the samples dispersed in the fixative solution to flow from the accommodation space 310 to the outside of the sample collection part 300.

Particularly, in a case where the sample inlet/outlet passage 360F is formed to be gradually narrowed in a direction from each of the sample inlets/outlets 360R1 and 360R2 to the inner side of the accommodation space 310, conversely, the sample inlet/outlet passage 360F is formed to be gradually expanded in a direction from the inner side of the accommodation space 310 to each of the sample inlets/outlets 360R1 and 360R2. Therefore, the sample accommodated in the accommodation space 310 is dispersed in the fixative solution, and it is possible to secure a certain amount or more of sample dispersion to the outside through the sample inlets/outlets 360R1 and 360R2.

As a result, the number of collected samples may be increased, and the samples accommodated in the accommodation space 310 are dispersed in the fixative solution to a certain degree or more, thereby allowing the samples to flow out of the collection part 300.

Further, the accommodation space 310 of the sample collection part 300 is preferably divided into two or more separate spaces, as follows.

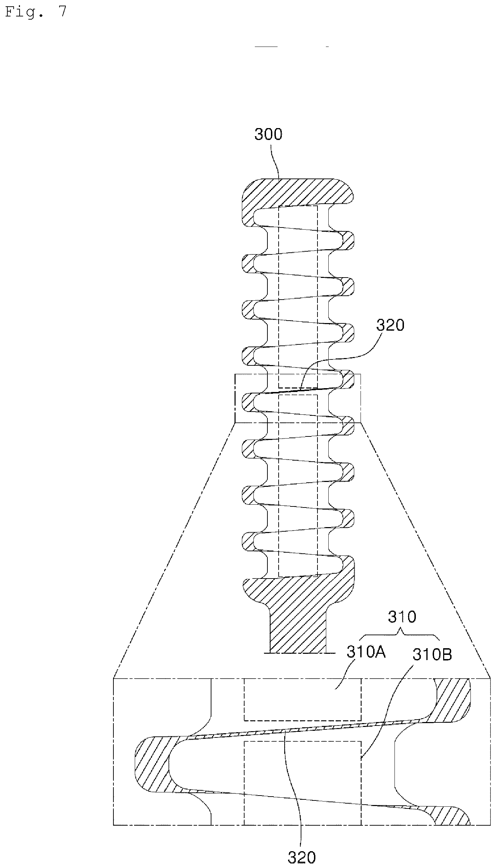

FIG. 7 is a side cross-sectional view schematically showing a modified sample collection part of the sample collection stick according to the embodiment of the present invention. As shown in FIG. 7, the sample collection part 300 preferably has one or more partition walls 320 provided therein and formed to spatially divide the accommodation space 310 into two or more spaces.

FIG. 7 is a view showing an example in which one partition wall 320 is provided. In this case, the accommodation space 310 is divided into two accommodation spaces 310A and 310B, and the two accommodation spaces 310A and 310B are separated from each other by one partition wall 320.

When one or more partition walls 320 are provided within the accommodation space 310, the viscous samples are deposited on the partition walls 320, which enables the surrounding samples to aggregate together. Therefore, it is possible to secure a certain number or more of collected samples by using one or more partition walls 320 and the separated accommodation spaces 310A and 310B.

In this manner, the partition wall 320 and the accommodation spaces 310A and 310B provided in the sample collection part 300 are preferably provided to enable a certain number or more of samples to aggregate together and to remain in the sample collection part 300.

In this manner, when one or more partition walls 320 are provided to divide the accommodation space 310 into a plurality of accommodation spaces, the number of samples separated from the accommodation space 310 formed in the sample collection part 300 is significantly reduced in the process of collecting the sample from the body, thereby increasing the number of collected samples. In addition, it is possible to increase the number of collected samples dissolved or dispersed in a reagent or a solution from the sample collection part 300 of the sample collection stick 10.

As described above, in the sample collection stick according to the embodiment of the present invention, the collected sample may be accommodated in the accommodation space of the sample collection part, and the sample introduced into the accommodation space is prevented from flowing to the outside of the accommodation space, thereby making it possible to further increase the number of collected samples as compared with a conventional sample collection swab.

Further, in the related art, a fiber layer is provided, but a fiber layer is not provided in the present invention. Accordingly, when the sample collection part is immersed into a fixative solution, the samples held or accommodated in the sample collection part may be easily dispersed in the fixative solution, thereby increasing the number of samples in the fixative solution.

As such, the sample collection stick according to the present invention does not require a flocking process required in the manufacturing process of an existing sample collection swab, thereby making it possible to reduce manufacturing costs of the sample collection stick. Additionally, since the sample collection stick does not have a fiber layer formed thereon, it is possible to solve a conventional problem that a fiber layer is separated from a conventional sample collection swab and remains in the body of a test subject.

Further, the sample collection stick according to the present invention increases the number of samples collected from the body of the test subject as compare with the conventional sample collection swab. In addition, it is possible to increase the number of samples dissolved or dispersed in a reagent or a solution from the sample collection stick. In this manner, there is an advantage in that accuracy of testing is improved with the increased number of collected samples.

In addition, the sample collection stick according to the present invention may shorten the time required for sample collection and may alleviate discomfort of the test subject during sample collection. Furthermore, since the sample collection part inserted into the oral cavity or the nasal cavity of the test subject is made of a polymer material or an elastomer material having flexibility and elasticity, thereby having an effect of alleviating discomfort of the test subject.

As described above, the present invention has been described through embodiments with reference to the accompanying drawings. However, since the above-described embodiments are only described as preferred embodiments of the present invention, the present invention should not be construed as being limited to the embodiments. The scope of the present invention should be defined in the appended claims and equivalents thereto.

### [Description of reference numerals]

10: Sample collection stick
100: Handle part
200: Support part
300: Sample collection part
310: Accommodation space 320: Partition wall
360R1, 360R2: Sample inlets/outlets

## Claims

1. A sample collection stick comprising:
a support part formed in a rod shape or a bar shape, the support part having a predetermined length; and
a sample collection part located at a front end of the support part and configured to collect a sample in contact with a body of a test subject,
wherein the sample collection part is formed in a cylindrical shape, and
wherein the sample collection part comprises:
an accommodation space formed in a longitudinal central axis of the sample collection part and configured to accommodate the sample therein; and
a plurality of sample inlets/outlets formed on an outer surface of the sample collection part and configured to communicate with the accommodation space, wherein the sample inlets/outlets are arranged in a row in a longitudinal direction of the sample collection part.

2. The sample collection stick according to claim 1, wherein the sample inlets/outlets are respectively arranged in a first row and a second row, wherein the first row and the second row are formed with the longitudinal central axis of the sample collection part interposed therebetween.

3. The sample collection stick according to claim 2, wherein the sample inlets/outlets in the first row and the sample inlets/outlets in the second row are alternately arranged in the longitudinal direction of the sample collection part.

4. The sample collection stick according to claim 1, wherein the sample collection part has one or more partition walls provided therein and formed to spatially divide the accommodation space into two or more spaces.

5. The sample collection stick according to claim 1, wherein the sample collection part has a sample inlet/outlet passage provided therein and formed to be narrowed or expanded in a direction traversing, from the sample inlets/outlets, the longitudinal central axis of the sample collection part.

6. The sample collection stick according to claim 1, wherein the sample collection part is made of a flexible material or an elastic material.

7. The sample collection stick according to claim 6, wherein the support part has a coupling jaw or a coupling protrusion formed to protrude from an outer surface thereof or a lid coupling groove formed in the outer surface, thereby enabling the outer surface of the support part to be fixedly coupled to a lid of a sample container configured to accommodate the sample collection part therein.
